# EUROPÄISCHE PATENTSCHRIFT

(11) **EP 0 907 636 B1**
(45) Veröffentlichungstag und Bekanntmachung des Hinweises auf die Patenterteilung: **25.10.2000**
(21) Anmeldenummer: 97929252.1
(22) Anmeldetag: 21.06.1997
(51) Int. Cl.: C07C 305/10, C11D 1/18

(54) **SULFATE VON FETTSÄUREPOLYHYDROXYALKYLAMIDEN UND IHRE VERWENDUNG**
SULPHATES OF FATTY-ACID POLYHYDROXYALKYLAMIDES AND THEIR USE
SULFATES DE POLYHYDROXYALKYLAMIDES D'ACIDE GRAS ET LEUR UTILISATION

(30) Priorität: 27.06.1996 DE 19625711
(43) Veröffentlichungstag der Anmeldung: 14.04.1999
(73) Patentinhaber: Clariant GmbH, 65929 Frankfurt am Main (DE)
(72) Erfinder: PAPENFUHS, Bernd, D-63179 Oberhausen (DE)
(86) Internationale Anmeldenummer: EP9703266
(87) Internationale Veröffentlichungsnummer: WO9749678

(56) Entgegenhaltungen:
- US-A- 2 717 894
- US-A- 5 312 934

## Beschreibung

Die Erfindung betrifft stark schäumende Sulfate von Fettsäurepolyhydroxyalkylamiden, Kompositionen mit diesen Sulfaten, ein Verfahren zur Herstellung der Sulfate und der Kompositionen und deren Verwendung.

Organische Sulfate, die lange hydrophobe Alkylgruppen enthalten, wie zum Beispiel Fettalkoholsulfate oder sulfatierte Fettalkoholalkoxylate, stellen wirksame und vielseitig verwendbare anionische Tenside dar.

In US-A 2 717 894 und WO-A 94/12472 werden Sulfate von Fettsäurepolyhydroxyalkylamiden beschrieben. Die Herstellung dieser Verbindungen erfolgt durch Umsetzung von Fettsäurepolyhydroxyalkylamiden mit typischen Sulfatier-Reagentien, wie Oleum, gasförmigem Schwefeltrioxid, SO₃/Pyridin-Komplex oder Chlorsulfonsäure. Wie in US-A 2 717 894 ausgeführt wird, weisen die sulfatierten Produkte verglichen mit den zugrundeliegenden neutralen Amiden ein deutlich erhöhtes Schaumvermögen auf.

Es wurde nun überraschenderweise gefunden, daß auch Sulfate von aminfunktionalisierten Fettsäurepolyhydroxyalkylamiden, die in nicht neutralisierter Form vorwiegend in zwitterionischer Struktur (siehe Beispiel) vorliegen, gegenüber den nichtionischen Ausgangsamiden erheblich stärker schäumen und sich zusätzlich durch eine verbesserte Hautverträglichkeit auszeichnen. Die Herstellung dieser neuen Wirkstoffe erfolgt mit Hilfe der bereits genannten Sulfatier-Reagentien.

Die erfindungsgemäßen Tenside entsprechen den nachstehenden Formeln (I) und (II) 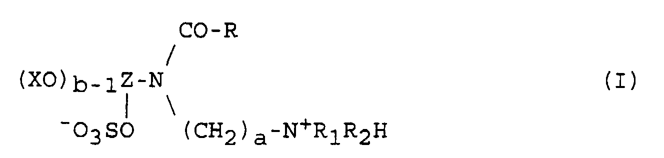 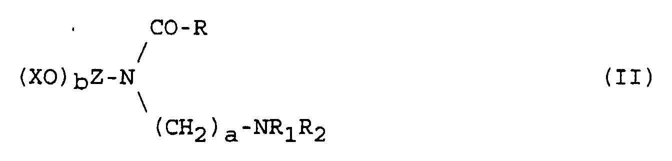 worin bedeuten
- CO-R: einen aliphatischen Acylrest mit 6 bis 22 Kohlenstoffatomen,
- R₁ und R₂: einen C₁ bis C₄-Alkylrest,
- a: eine Zahl von 2 bis 4,
- b: eine Zahl von 3 bis 9 und
- Z(OX)_{b}: einen unverzweigten, aliphatischen, partiell sulfatierten Polyhydroxykohlenwasserstoffrest mit 4 bis 12 Kohlenstoffatomen im Rest Z, wobei X ein Wasserstoffatom oder eine SO₃H-Gruppe beziehungsweise eine SO₃M-Gruppe ist, worin M ein Alkalibeziehungsweise ein halbes Erdalkalimetalläquivalent oder Ammonium bedeutet, mit der Maßgabe, daß X ein- bis (b-2)mal ungleich Wasserstoff ist.

Die erfindungsgemäßen Sulfate der Formeln (I) und (II), beispielsweise die im Beispiel beschriebene Verbindung 2, ergeben in 0,1%iger Konzentration in Wasser bei pH 7 sehr gute Werte im Ross-Miles-Schaumhöhetest und im Schlagschaum-Volumentest. Die Sulfate gemäß den Formeln (I) und (II) lassen sich beliebig mit kationischen, nichtionischen, amphoteren oder anionischen Tensiden kombinieren, beispielsweise mit Ethersulfaten auf Basis von Glykolmonoalkylethern oder Alkylsulfaten beziehungsweise Alkylsulfonaten. Die Sulfate gemäß den Formeln (I) und (II) können auch in Kombination mit Fettsäurepolyhydroxylamiden, insbesondere mit den zu ihrer Herstellung verwendeten N,N-Dialkylaminoalkylzuckeramiden, vorteilhaft verwendet werden. Die erfindungsgemäßen Tenside lösen sich in Wasser, niederen Alkoholen, niederen Glykolen oder einer Mischung dieser Lösemittel. Gleiches gilt für Kompositionen der Sulfate gemäß den Formeln (I) und (II) mit geeigneten Cotensiden.

Im folgenden wird zunächst die Herstellung eines geeigneten Dialkylaminopropyl-Glucamids gemäß einem bereits bekannten Verfahren beschrieben. Aus diesem Edukt werden im Beispiel neue Sulfate gemäß den Formeln (I) und (II) hergestellt.

Herstellung von Fettsäurepolyhydroxyalkylamid C_{12/14}-Dimethylaminopropyl-Glucamid (DMAP-GA):
304,5 g einer 41gew.%igen wäßrigen Lösung eines technischen N-(3-Dimethylamino)-propylglucamins (hergestellt nach US-A-4 021 539, Beispiel F), im folgenden DMAP-G, mit einem DMAP-G-Gehalt von 96 Gew.-%, bezogen auf Festsubstanz (das sind 0,45 mol DMAP-G), werden mit Hilfe einer üblichen Strippapparatur entwässert. Dazu wird die Lösung auf etwa 100 °C erhitzt und zunächst bei Normaldruck bis zu einem Restwassergehalt von etwa 5 Gew.-% entwässert. Die Lösung wird nun zur weiteren Entwässerung auf 130 °C erhitzt und bei dieser Temperatur gehalten, wobei schrittweise ein Vakuum von bis zu 25 mbar angelegt wird. Es wird bis zu einem Restwassergehalt von 0,2 Gew.-% entwässert. Zur so erhaltenen Schmelze werden bei 130 °C unter Rühren 104,1 g (0,473 mol) C_{12/14}-Fettsäuremethylester (Molmasse 220 g/mol, nach Verseifungszahl) gegeben. Die Mischung hat eine Temperatur von 120 °C. Nun werden noch 6,1 g Natriummethylat (30gew.%ig in Methanol, 7,5 Mol-%, bezogen auf DMAP-G) innerhalb von 3 Minuten zugetropft und die Reaktionsmischung bei etwa 100 °C gehalten. Das durch die Reaktion entstehende Methanol wird zunächst im System belassen. Nach etwa 20 Minuten liegt eine klare Reaktionslösung vor. Nun wird ein Vakuum von 60 mbar angelegt und eine Temperatur von circa 95 °C eingestellt, wobei die Reaktionsmischung etwa 80 Minuten unter Abdestillation des Methanols weiterreagiert. Die so erhaltene Lösung enthält circa 90 Gew.-% C_{12/14}-DMAP-GA, bezogen auf den Feststoffanteil.

### Beispiel

Herstellung von C_{12/14}-DMAP-GA-Sulfat (Verbindung 1):

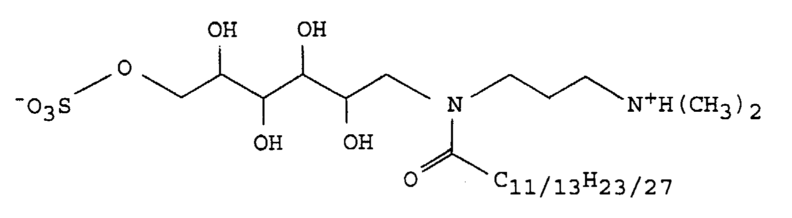

In einem Fallfilmreaktor wird ein 90 Gew.-% C_{12/14}-DMAP-GA enthaltender Reaktormix (Herstellung wie oben beschrieben) mit einem 2,2 Vol.-% SO₃ enthaltenden SO₃/Luft-Gemisch in einem Molverhältnis von 0,98 (SO₃ : DMAG-GA) zur Reaktion gebracht. Das abfließende Produkt mit einem pH-Wert von 2,2 (Messung in 1%iger wäßriger Lösung) wird in eine eisgekühlte Vorlage abgeführt. Der Gehalt an der erfindungsgemäßen Verbindung 1 beträgt 73,1 %.

Durch Neutralisation der Verbindung 1 mit wäßriger Natronlauge kann das erfindungsgemäße Natriumsalz (Verbindung 2) erhalten werden:

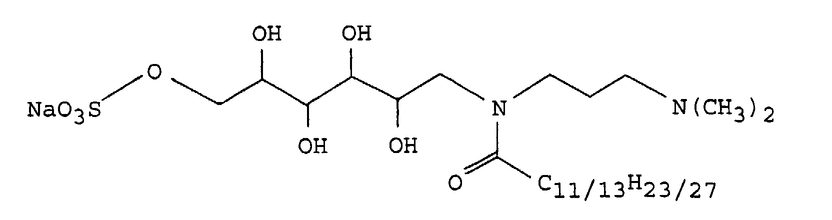

## Patentansprüche

1. Starkschäumende Tenside, gekennzeichnet durch die nachstehenden Formeln (I) und (II) 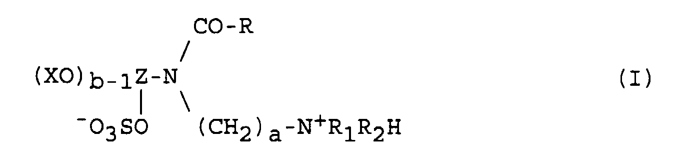 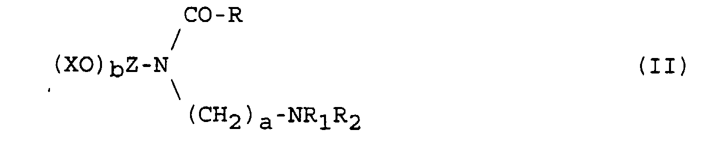 worin bedeuten
CO-R einen aliphatischen Acylrest mit 6 bis 22 Kohlenstoffatomen,
R₁ und R₂ einen C₁ bis C₄-Alkylrest,
a eine Zahl von 2 bis 4,
b eine Zahl von 3 bis 9 und
Z(OX)_{b} einen unverzweigten, aliphatischen, partiell sulfatierten Polyhydroxykohlenwasserstoffrest mit 4 bis 12 Kohlenstoffatomen im Rest Z, wobei X ein Wasserstoffatom oder eine SO₃H-Gruppe beziehungsweise eine SO₃M-Gruppe ist, worin M ein Alkali- beziehungsweise ein halbes Erdalkalimetalläquivalent oder Ammonium bedeutet, mit der Maßgabe, daß X ein- bis (b-2)mal ungleich Wasserstoff ist.

2. Herstellung von Verbindungen der Formeln (I) und (II) durch Umsetzung von Verbindungen der nachstehenden Formel 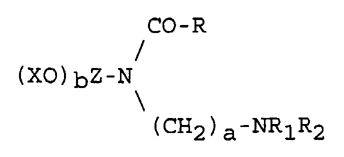 worin R, R₁, R₂, a und b die in Anspruch 1 genannte Bedeutung haben, Z(OX)_{b} ein unverzweigter aliphatischer Polyhydroxykohlenwasserstoffrest mit 4 bis 12 Kohlenstoffatomen im Rest Z und X Wasserstoff ist,
mit Sulfatier-Reagentien aus der Gruppe Oleum, Schwefeltrioxid, Schwefeltrioxid/Pyridin-Komplex oder Chlorsulfonsäure, wobei die primär erhaltenen Sulfate der Formel (I) durch Neutralisation in die entsprechenden Alkali-, Erdalkali- oder Ammoniumsalze der Formel (II) überführt werden können.

3. Verfahren nach Anspruch 2, wobei gasförmiges Schwefeltrioxid verwendet wird.

4. Verwendung von Verbindungen der Formeln (I) und/oder (II) als Wirkstoff in Waschmitteln und kosmetischen Formulierungen.

5. Kompositionen, gekennzeichnet durch einen Gehalt von mindestens 2 Gew.-%, bezogen auf die Komposition, von mindestens einer Verbindung gemäß Anspruch 1.

6. Kompositionen gemäß Anspruch 5, welche zusätzlich zu einem Tensid gemäß Anspruch 1 mindestens ein Cotensid aus der Gruppe der kationischen, nichtionischen, amphoteren und/oder anionischen Tenside enthalten.

7. Kompositionen gemäß den Ansprüchen 5 oder 6, dadurch gekennzeichnet, daß sie mindestens ein Lösungsmittel aus der Gruppe Wasser, niedere Alkohole, niedere Glykole oder einer Mischung davon enthalten.

## Claims

1. A high-foaming surfactant which has the following formula (I) or (II) 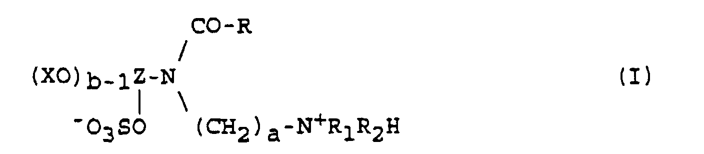 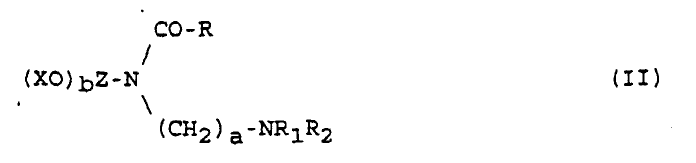 in which
CO-R is an aliphatic acyl radical having 6 to 22 carbon atoms,
R₁ and R₂ are a C₁ to C4-alkyl radical,
a is a number from 2 to 4,
b is a number from 3 to 9 and
Z(OX)_{b} is an unbranched, aliphatic, partly sulfated polyhydroxyhydrocarbon radical having 4 to 12 carbon atoms in the radical Z, where X is a hydrogen atom or an SO₃H group or an SO₃M group, in which M is one alkali metal or half an alkaline earth metal equivalent or ammonium, with the proviso that X is not hydrogen in one to (b-2) cases.

2. The preparation of a compound of the formula (I) or (II) by reaction of a compound of the following formula 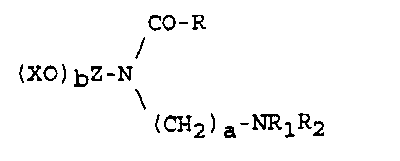 in which R, R₁, R₂, a and b have the meaning given in claim 1, Z(OX)_{b} is an unbranched aliphatic polyhydroxyhydrocarbon radical having 4 to 12 carbon atoms in the radical Z and X is hydrogen,
with a sulfating reagent from the group consisting of oleum, sulfur trioxide, sulfur trioxide/pyridine complex or chlorosulfonic acid, it being possible for the sulfate of the formula (I) primarily obtained to be converted into the corresponding alkali metal, alkaline earth metal or ammonium salt of the formula (II) by neutralization.

3. The process as claimed in claim 2, wherein gaseous sulfur trioxide is used.

4. The use of a compound of the formula (I) or (II) as an active compound in detergents and cosmetic formulations.

5. A composition which comprises a content of at least 2 % by weight, based on the composition, of at least one compound as claimed in claim 1.

6. A composition as claimed in claim 5, which, in addition to a surfactant as claimed in claim 1, comprises at least one cosurfactant from the group consisting of cationic, nonionic, amphoteric and anionic surfactants.

7. A composition as claimed in claim 5 or 6, which comprises at least one solvent from the group consisting of water, lower alcohols, lower glycols and mixtures thereof.

## Revendications

1. Agent tensioactif à fort pouvoir moussant, caractérisé par les formules (I) et (II) suivantes 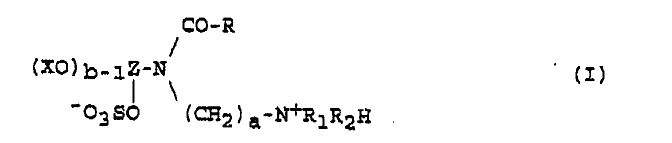 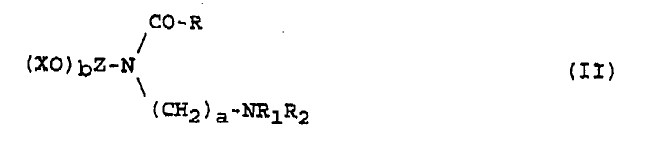 dans lesquelles
CO-R représente un groupe acyle aliphatique ayant de 6 à 22 atomes de carbone,
R₁ et R₂ représentent un groupe alkyle en C₁ à C₄,
a représente un nombre variant de 2 à 4,
b représente un nombre variant de 3 à 9 et
Z(OX)_{b} représente un résidu polyhydroxyhydrocarboné non ramifié, aliphatique, partiellement sulfaté ayant de 4 à 12 atomes de carbone dans le groupe Z, dans lequel X est un atome d'hydrogène ou un groupe SO₃H, respectivement un groupe SO₃M, dans lequel M représente un métal alcalin ou un demi-équivalent de métal alcalino-terreux ou l'ammonium, sous réserve que X soit différent d'un atome d'hydrogène de une à (b-2) fois.

2. Préparation de composés des formules (I) et (II) en mettant à réagir des composés de la formule suivante 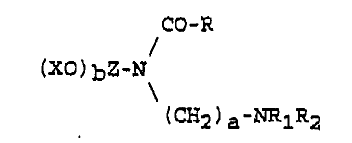 dans laquelle
R, R₁, R₂, a et b ont la signification indiquée à la revendication 1,
Z(OX)_{b} est un radical polyhydroxyhydrocarboné non ramifié aliphatique ayant de 4 à 12 atomes de carbone dans le groupe Z et
X est un atome d'hydrogène,
avec des réactifs de sulfatation choisis parmi l'oléum, le trioxyde de soufre, le complexe trioxyde de soufre / pyridine ou l'acide chlorosulfonique, les sulfates primaires de formule (I) obtenus pouvant être transformés par neutralisation en sels correspondants de métaux alcalins, de métaux alcalino-terreux ou d'ammonium de formule (II).

3. Procédé selon la revendication 2, dans lequel on utilise le trioxyde de soufre gazeux.

4. Utilisation de composés des formules (I) et/ou (II) comme substance active dans des détergents et des formulations cosmétiques.

5. Composition, caractérisée par une teneur d'au moins 2% en poids, par rapport à la composition, d'au moins un composé selon la revendication 1.

6. Composition selon la revendication 5, qui contient en plus d'un agent tensioactif selon la revendication 1, au moins un co-tensioactif choisi parmi les agents tensioactifs cationiques, non ioniques, amphotères et/ou anioniques.

7. Composition selon la revendication 5 ou 6, caractérisée en ce qu'elle contient au moins un solvant choisi parmi l'eau, les alcools de bas poids moléculaire, les glycols de bas poids moléculaire ou un mélange de ceux-ci.
